(19) Europäisches Patentamt
European Patent Office
Office européen des brevets

(11) Publication number: 0 233 040 A2

(12) # EUROPEAN PATENT APPLICATION

(21) Application number: 87300947.6

(22) Date of filing: 03.02.87

(51) Int. Cl.4: C 12 N 5/00, C 12 P 7/24, C 12 P 19/44, C 12 P 7/44

(30) Priority: 04.02.86 JP 22503/86
06.06.86 JP 131344/86

(43) Date of publication of application: 19.08.87 Bulletin 87/34

(84) Designated Contracting States: ES FR IT

(71) Applicant: AJINOMOTO CO., INC., 5-8, Kyobashi 1-chome, Chuo-ku, Tokyo 104 (JP)

(72) Inventor: Sano, Konosuke Central Research Laboratories, Ajinomoto Co., Inc. No. 1-1, Suzuki-cho, Kawasaki-ku Kawasaki-shi Kanagawa-ken (JP)
Inventor: Himeno, Hyota Central Research Laboratories, Ajinomoto Co., Inc. No. 1-1, Suzuki-cho, Kawasaki-ku Kawasaki-shi Kanagawa-ken (JP)

(74) Representative: Bond, Bentley George et al, HASELTINE LAKE & CO. Hazlitt House 28 Southampton Buildings Chancery Lane, London, WC2A 1AT (GB)

(54) **Stigma of crocus sativus L. and method for the production thereof.**

(57) The present invention relates to a method for the production of a stigma of Crocus sativus L. and the corresponding organs in vitro, the produced organs and a method for the production of safranal, crocin, crocetin and derivatives thereof in the said organs.

EP 0 233 040 A2

# STIGMA OF CROCUS SATIVUS L. AND METHOD FOR THE PRODUCTION THEREOF

The present invention relates to a method for the production of a stigma of Crocus sativus L. and the corresponding organs in vitro, the produced organs and a method for the production of safranal, crocin, crocetin and derivatives thereof in the said organs.

Crocus sativus L. is a perennial plant belonging to Iridaceae. A product obtained from this plant by collecting and drying the stigma thereof, which is called saffron (Spanish saffron or Safran), has long been used in medicines, spices and coloring materials. Saffron was used at least before 1700 B.C., and is still widely used as a material for peptics, tonics, lenitives and other similar medicines, or as additives for various cooking materials, for example, for flavoring or coloring butter, cheese, liqueur, ice cream and the like, or as a coloring material for lipstick or rouge. Saffron is extremely valuable and high-priced.

*Crocus sativus* L. is said to be native to South Europe and Asia Minor, and grows in temperate and subtropical regions. However, the production of saffron is carried out in relatively limited regions, and the main producing center is Spain. The reason is considered to mainly depend upon the quality of the product. *Crocus sativus* L. has been widely cultivated in Europe, India and China for self-sufficiency during periods when distribution was interrupted because of political reasons.

The method for production of saffron comprises extracting the stigma of *Crocus sativus* L. within a few days after the plants have bloomed and drying them; The season is only once a year, and the operation is carried out entirely by hand. Thus, the agricultural production of saffron is low-productive, which is considered to be the main reason for the high price of saffron. In order to intensify the productivity to increase the yield of saffron, mass-propagation of the plants is required. However, *Crocus sativus* L. does not yield any seeds and multiplies by bulbs. The propagation of bulbs of *Crocus sativus* L. is not easy. Thus, the propagativity is low, which is considered to be another reason for the high price of saffron.

Recently, a study has been carried out, where a callus is formed from the Crocus sativus L. by tissue culture, and crocin, which is a yellow pigment, is produced by the callus. (Refer to Japanese Patent Application OPI No. 115181/82. The term "OPI" as used herein means an "unexamined and published application".) However, it is difficult to obtain the same fragrance or flavor by use of the callus as compared to the above-described method, and the callus will not be sufficient as a substitute for saffron.

Nowadays, the techniques for the cultivation of organs, tissues and cells of plants have been highly developed, and the industrial production of useful substances from a variety of plants in vitro has become possible. Nevertheless, such techniques have not heretofore been applied to enhancing the production of saffron by way of in vitro culturing, growing, and maturing of a stigma of Crocus sativus L.

Accordingly, a need therefore continues to exist for new methods for enhancing the production of saffron from Crocus sativus L.

Accordingly, it is an object of the present invention to provide a method for in vitro culturing, growing, and maturing a stigma of Crocus sativus L.

It is another object of the present invention to provide a method for in vitro culturing, growing, and maturing in a synthetic culture medium of the corresponding organs of a stigma of Crocus sativus L.

It is another object of the present invention to provide a method for the production of saffronal, picrocrocin or derivatives thereof, and crocin, crocetin and derivatives thereof.

According to the present invention the foregoing and other objects are attained by the method described more precisely as follows:

A gynoecium as excised from flower buds of Crocus sativus L., a stigma-like structure as propagated in a synthetic culture medium, or an unmatured gynoecium (or an ovary of a stigma) as excised from flower buds of Crocus sativus L. under sterile conditions is implanted in a solid or liquid synthetic culture medium for cultivation of plant tissues and kept in a dark place, a light place, or in an alternating dark and light place at 15 to 40°C, whereby a great number of stigma-like organs propagate or significantly grow and mature under conditions of the presence of a pertinent combination and concentration of hormones. In this process, the addition of hormones is the most important factor, and the combination of an auxin and a cytokinin with an appropriate concentration ratio is necessary to attain the best effect.

In order to grow and mature a stigma of *Crocus sativus* L. or the corresponding organs in a synthetic culture medium, it is desired to add preferably 0.01 to 20 ppm, and more preferably 1 to 5 ppm of cytokinin to the synthetic culture medium.

In order to proliferate and propagate the stigma of *Crocus sativus* L. or the corresponding organs in a synthetic culture medium, it is desirable to add cytokinin and auxin to the synthetic culture medium each in an amount of preferably 0.01 to 20 ppm, and more preferably 1 to 5 ppm of cytokinin and 0.1 to 20 ppm of auxin. Substances which can be used as an auxin include, for example, indole-acetic acid, indole-propionic acid, indole-butyric acid, α-naphthalene-acetic acid, β-naphthoxy-acetic acid, methyl indole-acetate, 4-chlorophenoxy-acetic acid, 2,4-dichlorophenoxy-acetic acid (2,4-D), 2-methyl-4-chlorophenoxy acid, 2,3,6-trichlorobenzoic acid, cis-citric acid and 2,4,5-trichloro-phenoxy-acetic acid (2,4,5-T); and substances which can be used as a cytokinin include, for example, cis-zeatin, trans-zeatin, ribosyl-zeatin and other similar zeatins, kinetin, senylaminopurine, benzyl-aminopurine and other similar kinetins, and benzylaminobenzimidazole, 6-benzyladenine, N-4-pyridyl-N'-phenylurea, N-2-chloro-4-pyridyl-N'-phenylurea and other similar pyridylphenylureas. These are added singly or in the form of a combination thereof as an auxin or a cytokinin.

Conventional media for plant tissue-culture (some are commercial products) can be used as the synthetic culture media to which the said hormones) is (are) added. For instance, Murashige and Skoog's medium, White's medium, Gamborg et al.'s medium, Nitsch's medium, Heller's medium, Schenk and Hildebrandt's medium, Nitsch and Nitsch's medium, Kohlenbach and Schmidt's medium, Knop's medium and Linsmaier and Skoog's medium as well as other media containing other carbon sources than sucrose and glucose and other nitrogen sources than ammonium salts and nitrates can be used. In addition, yeast extract, casamino acid, peptone, amino acid powder, coconut milk, ascorbic acid, extract of Crocus sativus L. plant body (e.g. bulbs, stems, leaves, etc.) may optionally be added thereto.

Saffron stigma-like organs as obtained in the synthetic culture medium to which cytokinin has been added can be cultured in the same culture condition or in a culture condition which is suitable for further growth and maturation of the said organs. Only the stigma or the corresponding organs obtained in synthetic culture containing both a cytokinin and an auxin or a cytokinin only can be used, so that these are directly dried or useful substances are extracted

therefrom, and if necessary, these may be transplanted for further maturation.

A fragrant component such as safranal and a yellow dye such as crocin, which are useful substances contained in these artificially cultured organs, can be used directly in the same form as a natural stigma, or these may be subjected to extraction in a conventional manner or may further be purified for practical use.

For identification and determination of these substances, gas chromatography or high speed-liquid chromatography can be used. As a result of such instrumental analysis, the existence of safranal and crocin and related derivatives such as picrocrocin and 2,6,6-trimethyl-4-hydroxy-1-cyclohexane-1-carboxyaldehyde were confirmed. All of these substances are those which are contained in a natural stigma. In addition, crocetin can easily be obtained from crocin by drying a natural stigma or by deglycosylating the same by chemical treatment or enzyme treatment.

Structural formulae of these substances are given below.

Safranal

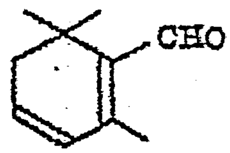

Picrocrocin

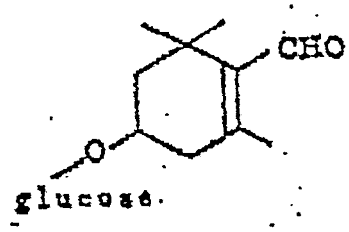

2,6,6-trimethyl-4-hydroxy-1-cyclohexane-1-carboxyaldehyde

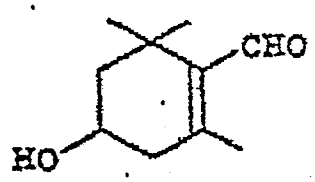

Crocin

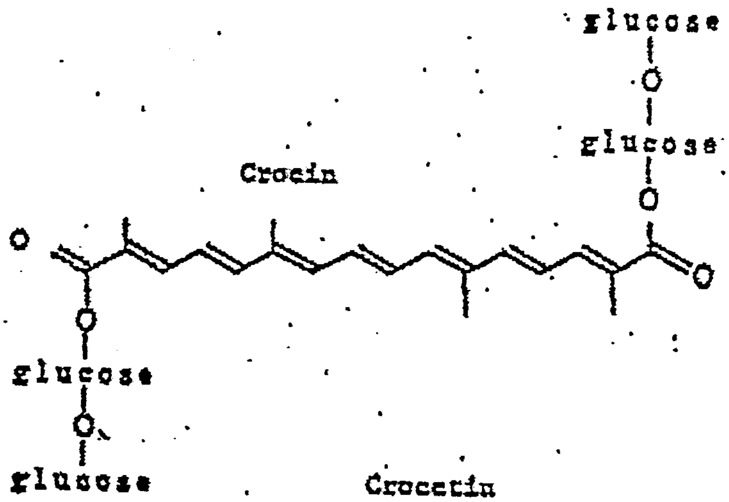

Crocetin

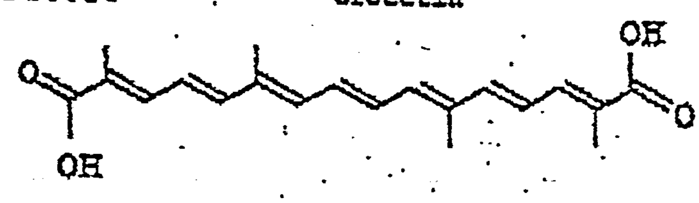

The invention now being generally described, the same will be better understood by reference to certain specific examples which are included herein for purposes of illustration only and are not intended to be limiting of the invention or any embodiment thereof, unless specified.

Example 1

A bulb (weight: about 30 g) of Crocus sativus L., which is a species for cultivation for the production of saffron, was germinated, the buds were excised, and the tunica thereof were sterilized with 1% sodium chlorite solution, washed with water, and then, the buds were cut to extract the gynoecium or the part containing the same therefrom. The gynoecium contained a stigma and an ovary. The stigma and/or ovary was (were) implanted on a solid Linsmaier and Skoog's medium (pH 5.8; 1% agar) to which 1 mg/l of benzyladenine had been added, and cultivated in the dark at 20°C. After one month, a stigma which had grown to a fresh weight of about 30 times that of the original weight was obtained. The stigma was colored yellow to deep orange and was confirmed to be useful for medicines, spices and coloring materials. The analytical result is given in the following Table 1.

The Linsmaier and Skoog's medium as used herein contains 370 mg of $MgSO_4.7H_2O$, 440 mg of $CaCl_2.2H_2O$, 1900 mg of $KNO_3$, 1650 mg of $NH_4NO_3$, 170 mg of $KH_2PO_4$, 27.8 mg of $FeSO_4.7H_2O$, 37.3 mg of $Na_2EDTA$, 22.3 mg of $MnSO_4.4H_2O$, 8.6 mg of $ZnSO_4.7H_2O$, 0.025 mg of $CuSO_4.5H_2O$, 0.025 mg of $CoCl_2.6H_2O$, 0.83 mg of KI, 6.2 mg of $H_3BO_3$, 0.25 mg of $Na_2MoO_4.2H_2O$, 30,000 mg of sucrose, 100 mg of myoinositol and 0.4 mg of thiamine hydrochloride per one liter.

Example 2

An ovary, which is a part of a gynoecium as extracted under sterile conditions in the same manner as in Example 1, was cultured in a medium containing both auxin and cytokinin, or more precisely, a Linsmaier and Skoog's medium containing 10 mg/l of α-naphthalene-acetic acid and 1 mg/l of kinetin, and a number of stigma-like structures were formed in 30 to 60 days. These newly formed organs were transplanted into a Linsmaier and Skoog's medium containing 5 mg/l of kinetin and cultivated therein for 2 months further, whereby they grew and matured to about 30 times the initial weight. The analytical result of these organs is given in Table 1.

Example 3

Stigma-like neoplastic organs obtained in the same manner as in Example 2 were cultivated for 2 months further in a Linsmaier and Skoog's medium containing 5 mg/l of kinetin and 0.1 mg/l of α-naphthalene-acetic acid, whereby these grew, hypertropied and matured to about 20 times the original weight. The analytical result of these organs is given in Table 1.

Table 1

Safranal, crocin and the derivatives thereof as produced by *in vitro* cultivated stigma and stigma-like organ

| Specimens | Safranal (ppm) | Picocrocin (ppm) | 2,6,6-trimethyl-4-hydroxy-1-cyclohexane-1-carboxyaldehyde | Crocin (ppm) |
|---|---|---|---|---|
| Example 1 | 5.5 | 640 | 8 | 615 |
| 2 | 2 | 452 | 10 | 731 |
| 3 | 3 | 539 | 64 | 925 |
| 10 | 1.5 | 1700 | 2 | 10800 |
| 11 | 2 | 260 | 8 | 1100 |

Example 4

In a similar experiment as Example 1, the auxin was replced by 0.1 mg/ml of α-naphthalene-acetic acid and the cytokinin by 5 mg/l of kinetin, and as a

result, about 10 stigma-like structures were formed from a half ovary in about 60 days.

Example 5

In a similar experiment as Example 1, the auxin was replaced by 1 mg/l of α-naphthalene-acetic acid and the cytokinin by 5 mg/l of kinetin, and as a result, four stigma-like organs were formed from a single stigma (the natural flower has three branches of stigma) in about 60 days.

Example 6

In a similar experiment as Example 1, the auxin was replaced by 10 mg/l of α-naphthalene-acetic acid and the cytokinin by 1 mg/l of benzyladenine, and as a result, about 30 stigma-like organs were formed from a half ovary in about 60 days.

Example 7

In a similar experiment as Example 1, the auxin was replaced by 10 mg/l of α-naphthalene-acetic acid and the cytokinin by 5 mg/l of benzyladenine, and as a result, about 10 stigma-like organs were formed from one ovary and one stigma in about 60 days.

Example 8

In a similar manner as Example 1, the auxin was replaced by 10 mg/l of indole-acetic acid and the cytokinin by 5 mg/l of benzyladenine, and as a result, 3 stigma-like organs were formed from a half ovary in about 60 days.

Example 9

In a similar experiment as Example 1, the auxin was replaced by 1 mg/l of α-naphthyl-acetic acid and the cytokinin by 1 mg/l of kinetin, and as a result, 10 stigma-like structures were formed from one branch of stigma.

Example 10

In a similar experiment as in Example 1, Linsmaier and Skoog's medium was replaced by Nitsch and Nitsch medium, and 1 ppm of α-naphthyl-acetic acid and 1 ppm kinetin were supplemented to it. After 30-60 days incubation, many (more than five) stigma-like structure (organs) were formed from a half ovary. The contents of secondary metabolites in one stigma-like structure are shown in Table 1.

Example 11

In a similar experiment as in Example 1, Gamborg B5 medium was used as the basal medium and 10 ppm α-naphthyl-acetic acid and 1 ppm of kinetin were added. After 30-60 days incubation, many (more than ten) stigma-like structure (organ) were formed from a half ovary. The contents of secondary metabolites in one stigma-like structure are shown in Table 1.

Example 12

In a similar experiment as shown in Example 1, auxin was replaced by 10 mg/l of α-indole-lactic acid, and cytokinin was replaced by 10 mg/l of benzyladenine. After 60 days incubation, 30 stigma-like structure (organs) were formed from a half ovary.

Obviously, numerous modifications and variations of the present invention are possible in light of the above teachings. It is therefore to be understood that within the scope of the appended claims, the invention may be practiced otherwise than as specifically described herein.

CLAIMS

1. A method for *in vitro* production of a stigma of *Crocus sativus* L., or the corresponding organs, which comprises culturing, growing, and maturing a stigma or corresponding organs in a synthetic culture medium containing a cytokinin.

2. A stigma of *Crocus sativus* L. or the corresponding organs, produced by culturing, growing, and maturing a stigma of *Crocus sativus* L. or the corresponding organs in a synthetic culture medium containing a cytokinin.

3. A method for *in vitro* proliferating and growing a stigma of *Crocus sativus* L. or the corresponding organs which comprises culturing a stigma of *Crocus sativus* L. or the corresponding organs in a synthetic culture medium containing a cytokinin and an auxin.

4. A stigma of *Crocus sativus* L. or the corresponding organs produced by proliferating and growing a stigma of *Crocus sativus* L. or the corresponding organs in a synthetic culture medium containing a cytokinin and an auxin.

5. A method for the production of safranal, picrocrocin or derivatives thereof, or crocin, crocetin or the derivatives thereof, which comprises excising a young stigma of *Crocus sativus* L. and cultivating said

stigma in a synthetic culture medium containing a cytokinin to produce one or more substances selected from the group consisting of safranal, picrocrocin and the derivatives thereof, crocin, crocetin and the derivatives thereof, and isolating one or more of said substances.

6. A method for the production of safranal, picrocrocin or derivatives thereof, or crocin, crocetin or the derivatives thereof, which comprises cultivating an *in vitro* proliferated stigma-like structure of *Crocus sativus* L. in a synthetic culture medium containing a cytokinin and an auxin to produce one or more substances selected from the group consisting of safranal, picrocrocin and derivatives thereof, crocin, crocetin and derivatives thereof; and isolating one or more of said substances.